# EUROPÄISCHE PATENTSCHRIFT

(11) **EP 1 682 559 B1**
(45) Veröffentlichungstag und Bekanntmachung des Hinweises auf die Patenterteilung: **30.12.2009**
(21) Anmeldenummer: 04790949.4
(22) Anmeldetag: 28.10.2004
(51) Int. Cl.: C07F 9/48, C07F 9/12, B01J 31/18

(54) **STERISCH ANSPRUCHSVOLLE CHELATPHOSPHINITPHOSPHITLIGANDEN, KATALYSATOR, UMFASSEND WENIGSTENS EINEN NICKEL(0)KOMPLEX STABILISIERT DURCH DIESEN LIGANDEN SOWIE EIN VERFAHREN ZUR HERSTELLUNG VON NITRILEN**
STERICALLY HINDERED CHELATE PHOSPHINITE-PHOSPHITE LIGAND, CATALYST, COMPRISING AT LEAST ONE NICKEL(0) COMPLEX STABILIZED BY SAID LIGAND AND METHOD FOR PRODUCTION OF NITRILES
LIGAND PHOSPHITE-PHOSPHINITE CHELATE A ENCOMBREMENT STERIQUE, CATALYSEUR COMPRENANT AU MOINS UN COMPLEXE DE NICKEL (0) STABILISE PAR CE LIGAND ET PROCEDE DE PRODUCTION DE NITRILES

(30) Priorität: 30.10.2003 DE 10350999
(43) Veröffentlichungstag der Anmeldung: 26.07.2006
(73) Patentinhaber: BASF SE, 67056 Ludwigshafen (DE)
(72) Erfinder: BARTSCH, Michael, 67433 Neustadt (DE); BAUMANN, Robert, 68159 Mannheim (DE); HADERLEIN, Gerd, 67269 Grünstadt (DE); FLORES, Miguel Angel, E-28300 Aranjuez (Madrid) (ES); JUNGKAMP, Tim, B-2950 Kapellen (BE); LUYKEN, Hermann, 67069 Ludwigshafen (DE); SCHEIDEL, Jens, 69493 Hirschberg (DE); SIEGEL, Wolfgang, 67117 Limburgerhof (DE)
(86) Internationale Anmeldenummer: PCT/EP2004/012176
(87) Internationale Veröffentlichungsnummer: WO 2005/042547

(56) Entgegenhaltungen:
- US-A- 5 523 453
- US-A- 5 693 843

## Beschreibung

Die vorliegende Erfindung betrifft neue Phosphinitphosphite, insbesondere Chelatphosphinitphosphite, und ein Verfahren zu ihrer Herstellung. Weiterer Gegenstand der vorliegenden Erfindung sind ihre Verwendung als Ligand in Übergangsmetallkomplexen, neue Übergangsmetallkomplexe und entsprechende Verfahren zu ihrer Herstellung. Des weiteren betrifft die vorliegende Erfindung die Verwendung der Obergangsmetallkomplexe als Katalysator und Verfahren in Gegenwart solcher Übergangsmetallkomplexe als Katalysator.

Chelatphosphinitphosphite, Nickel-Komplexe mit solchen Phosphinitphosphiten als Liganden und die Verwendung solcher Komplexe als Katalysator sind bekannt.

US 5,693,843 und 5,523,453 beschreiben Verfahren zur Hydrocyanierung von ungesättigten organischen Verbindungen und die Isomerisierung von Nitrilen in Anwesenheit von Nickel(0)-Komplexen mit Chelatphosphinitphosphiten als Ligand. Wünschenswert ist eine Verbesserung der Stabilität der Chelatphosphinitphosphit -Liganden zur Erhöhung der Standzeit des Katalysators. Weiterhin ist eine Verbesserung der Selektivität des Katalysators, beispielsweise bei der Hydrocyanierung von Butadien hinsichtlich 3-Pentennitril oder bei der Hydrocyanierung von 3-Pentennitril hinsichtlich Adipodinitril, und eine Verbesserung der Raum-Zeit-Ausbeute wünschenswert.

Die Aufgabe der vorliegenden Aufgabe war es somit, als Chelatphosphinitphosphit geeignete Phosphinitphosphite bereitzustellen, die die Hydrocyanierung von ungesättigten organischen Verbindungen bei hoher Stabilität, hoher Reaktivität und hoher Selektivität des Katalysators auf technisch einfache und wirtschaftliche Weise ermöglichen.

Demgemäß wurden Phosphinitphosphite I der Formel 1 oder 2 oder 3 oder 4 oder 5 oder 6 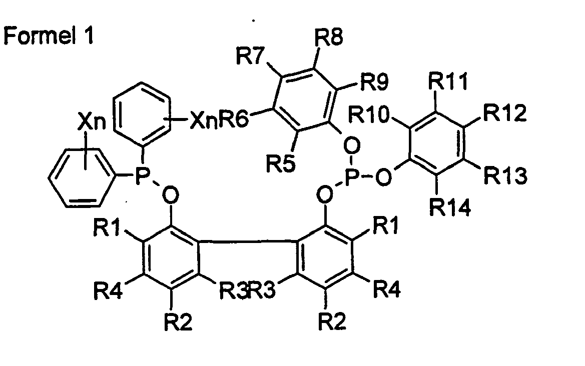 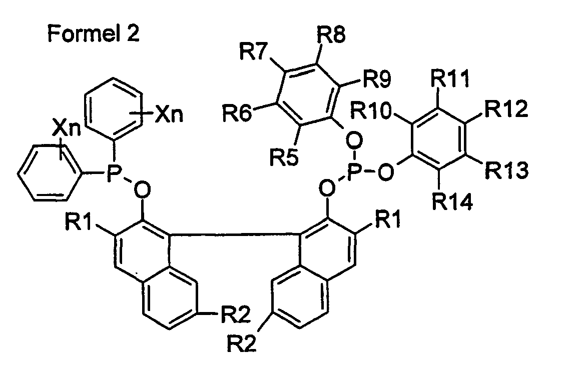 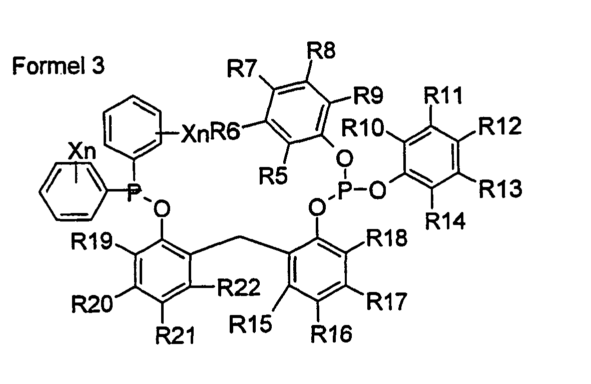 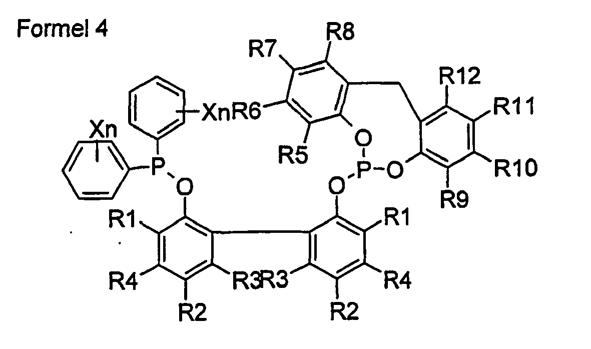 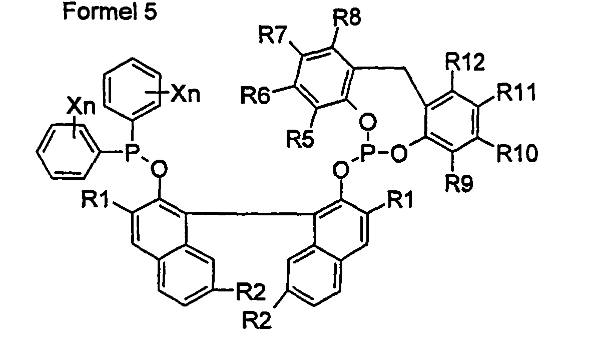 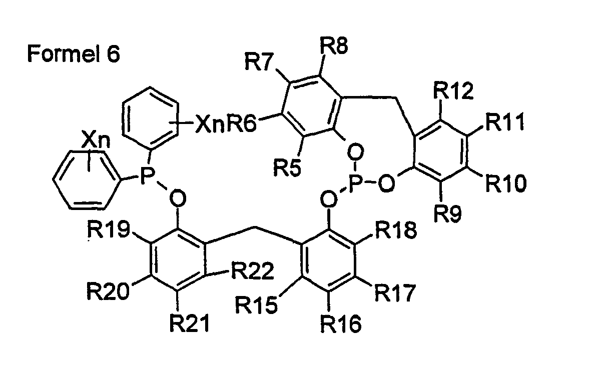 mit
R1, R2, R4 sind unabhängig voneinander H, eine Alkylgruppe mit 1 bis 8 Kohlenstoffatomen, mit der Maßgabe; dass mindestens eine der Gruppen R1, R2, R4 ungleich H ist,
R5 bis R22 sind unabhängig voneinander H, eine Alkylgruppe mit 1 bis 8 Kohlenstoffatomen,
R3 ist H, Methyl oder Ethyl,
X ist F, Cl oder CF₃, wenn n gleich 1 oder 2 ist,
X ist H, wenn n gleich 0 ist.
und deren Gemische
gefunden.

Erfindungsgemäß sind die Reste R1, R2, R4 unabhängig voneinander eine Alkyl gruppe mit 1 bis 8 Kohlenstoffatomen, mit der Maßgabe, dass mindestens eine der Gruppen R1, R2, R4 ungleich H ist,

Steht R1 für Wasserstoff, so können R2 Wasserstoff und R4 eine Alkyl gruppe mit 1 bis 8 Kohlenstoffatomen sein oder R2 eine Alkylgruppe mit 1 bis 8 Kohlenstoffatomen und R4 Wasserstoff sein oder R2 und R4 unabhängig voneinander eine Alkylgruppe mit 1 bis 8 Kohlenstoffatomen sein.

Steht R1 für eine Alkylgruppe mit 1 bis 8 Kohlenstoffatomen, so können R2 und R4 Wasserstoff sein oder R2 unabhängig von R1 eine Alkylgruppe mit 1 bis 8 Kohlenstoffatomen und R4 Wasserstoff sein oder R2 Wasserstoff und R4 unabhängig von R1 eine Alkylgruppe mit 1 bis 8 Kohlenstoffatomen sein oder R2 und R4 unabhängig voneinander und unabhängig von R1 eine Alkyl gruppe mit 1 bis 8 Kohlenstoffatomen sein.

Als Alkylgruppe mit 1 bis 8 Kohlenstoffatomen kommt vorzugsweise eine Alkylgruppe mit 1 bis 8 Kohlenstoffatomen, insbesondere 1 bis 4 Kohlenstoffatomen, vorteilhaft ausgewählt aus der Gruppe bestehend aus Methyl, Ethyl, n-Propyl, i-Propyl, n-Butyl, s-Butyl, i-Butyl und t-Butyl, insbesondere aus der Gruppe bestehend aus Methyl, Ethyl, n-Propyl, i-Propyl und t-Butyl, in Betracht.

Erfindungsgemäß stellt R3 H oder eine Methyl- oder eine Ethylgruppe dar.

Erfindungsgemäß können die mit einem Phosphoratom verbundenen Phenylgruppen unsubstituiert sein oder pro Phenylgruppe unabhängig voneinander 1 oder 2 Substituenten X tragen, so dass sich für n die Werte 0, 1 oder 2 ergeben.

Die beiden mit einem Phosphoratom verbundenen Phenylgruppen können dabei gleich oder unterschiedlich substituiert sein, wobei sich im Falle einer unterschiedlichen Substitution die Unterschiede sowohl auf die Zahl der Substituenten, wie auch auf die Art der Substituenten bezieht. Im Sinne der vorliegenden Erfindung umfassen die Formeln 1, 2 und 3 sowohl eine gleiche, wie auch eine unterschiedliche Substitution der mit einem Phosphoratom verbundenen Phenylgruppen.

Erfindungsgemäß ist X gleich F, Cl oder CF₃, vorzugsweise F oder CF₃. Im Fall von n gleich 2 können die beiden Reste X1 und X2 unabhängig voneinander F, Cl oder CF₃ darstellen, also F und F, F und Cl, F und CF₃, Cl und Cl, Cl und CF₃, CF₃ und CF₃, vorzugsweise F und F, CF₃ und CF₃.

In einer bevorzugten Ausführungsform kommt im Fall von n gleich 1 und X gleich F eine Substitution in m-Stellung zu dem mit dem Phenylring verbundenen Phosphoratom in einem mit einem Phosphoratom verbundenen Phenylring in Betracht.

In einer weiteren bevorzugten Ausführungsform kommt im Fall von n gleich 1 und X gleich CF₃ eine Substitution in p-Stellung zu dem mit dem Phenylring verbundenen Phosphoratom in einem mit einem Phosphoratom verbundenen Phenylring in Betracht.

In einer bevorzugten Ausführungsform kommt im Fall von n gleich 2 und X1 und X2 gleich F eine Substitution in den beiden m-Stellungen zu dem mit dem Phenylring verbundenen Phosphoratom in einem mit einem Phosphoratom verbundenen Phenylring in Betracht.

In einer weiteren bevorzugten Ausführungsform kommt im Fall von n gleich 2 und X1 und X2 gleich CF₃ eine Substitution in den beiden m-Stellungen zu dem mit dem Phenylring verbundenen Phosphoratom in einem mit einem Phosphoratom verbundenen Phenylring in Betracht.

Besonders bevorzugte Phosphinitphosphite sind solche der nachfolgenden Formeln la - Ij mit den in Tabelle 1 bezeichneten Bedeutungen der Gruppen R1, R2, R3 und R4, sowie R18 bis R22.

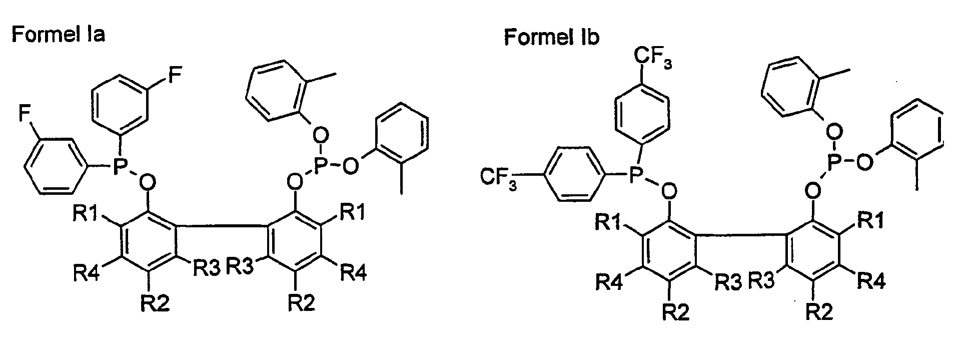

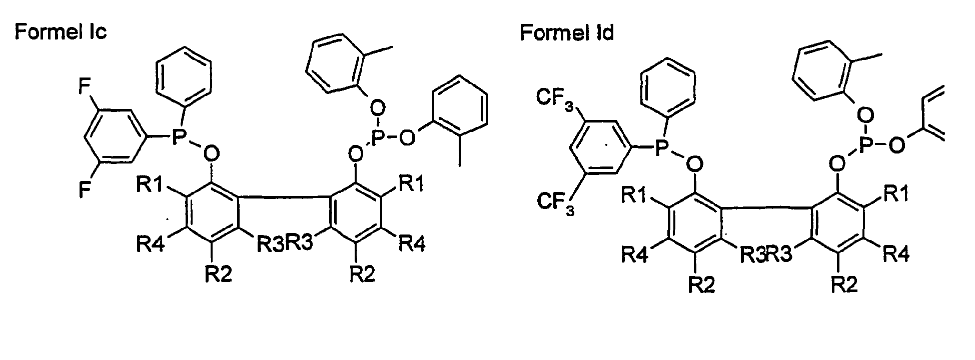

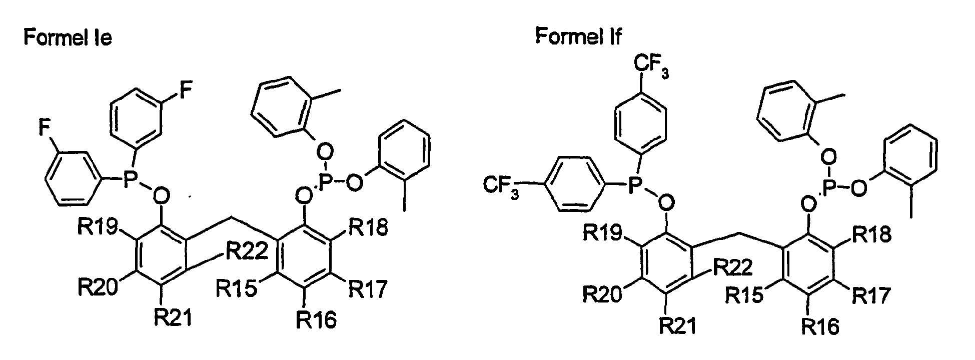

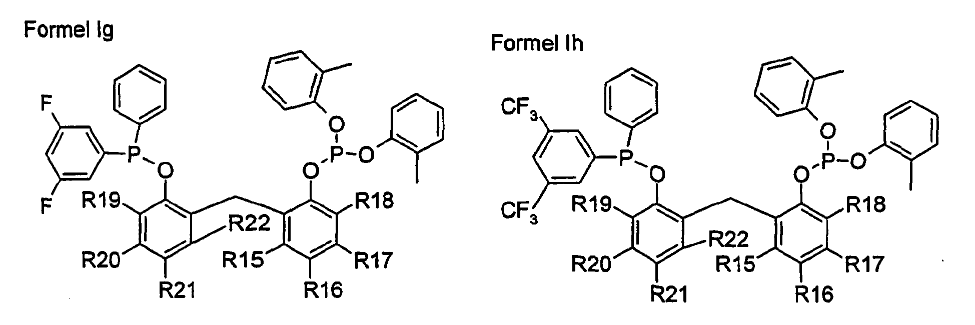

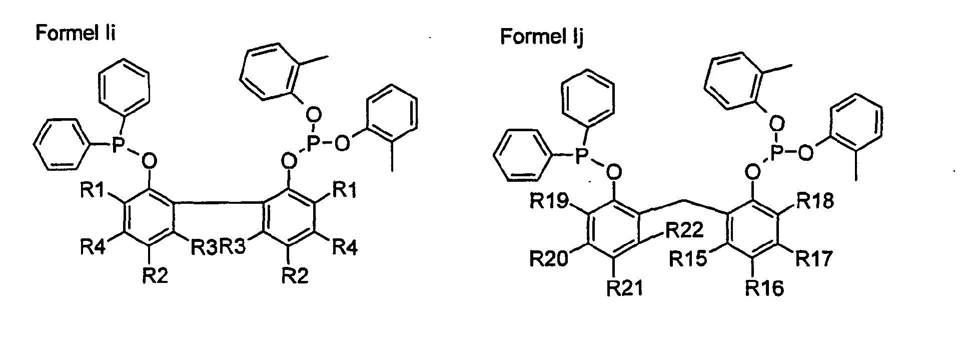

In diesen Formeln haben die Reste R1, R2, R3 und R4, sowie R18 bis R22 folgenden Bedeutungen:

**Tabelle 1**

| Formel | R1, R18, R19 | R2, R16, R21 | R3, R15, R22 | R4, R17, R20 |
|---|---|---|---|---|
| Ia1, Ib1, Ic1, Id1, Ie1, If1, Ig1, Ih1, Ii1, Ij1 | Me | Me | H | H |
| Ia2, Ib2, Ic2, Id2, Ie2, If2, Ig2, Ih2, Ii2, Ij2 | Et | t-Bu | H | H |
| Ia3, Ib3, Ic3, Id3, Ie3, If3, Ig3, Ih3, Ii3, Ij3 | i-Pr | H | Me | H |
| Ia4, Ib4, Ic4, Id4, Ie4, If4, Ig4, Ih4, Ii4, Ij4 | t-Bu | t-Bu | H | H |
| Ia5, Ib5, Ic5, Id5, Ie5, If5, Ig5, Ih5, Ii5, Ij5 | Et | Me | H | H |
| Ia6, Ib6, Ic6, Id6, Ie6, If6, Ig6, Ih6, Ii6, Ij6 | n-Pr | Me | H | H |
| Ia7, Ib7, Ic7, Id7, Ie7, If7, Ig7, Ih7, Ii7, Ij7 | t-Bu | Me | H | H |
| Ia8, Ib8, Ic8, Id8, Ie8, If8, IgB, Ih8, Ii8, Ij8 | Me | H | Me | Me |
| Ia9, Ib9, Ic9, Id9, Ie9, If9, Ig9, Ih9, Ii9, Ij9 | Me | t-Bu | H | H |

Weiterhin besonders bevorzugte Phosphinitphosphite sind solche der nachfolgenden Formeln Ik-Io.

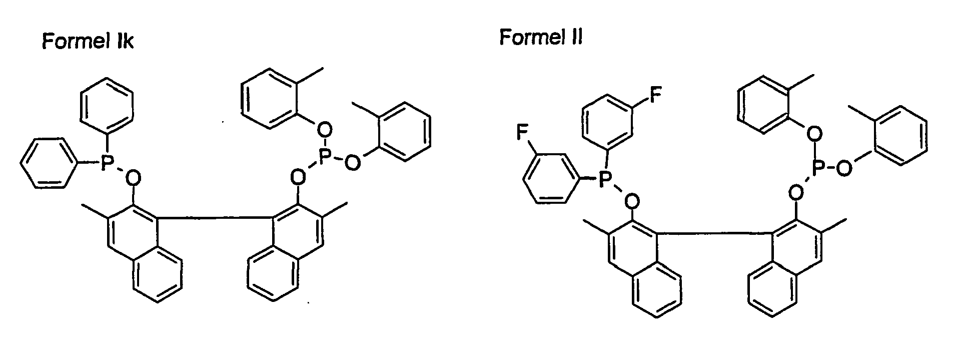

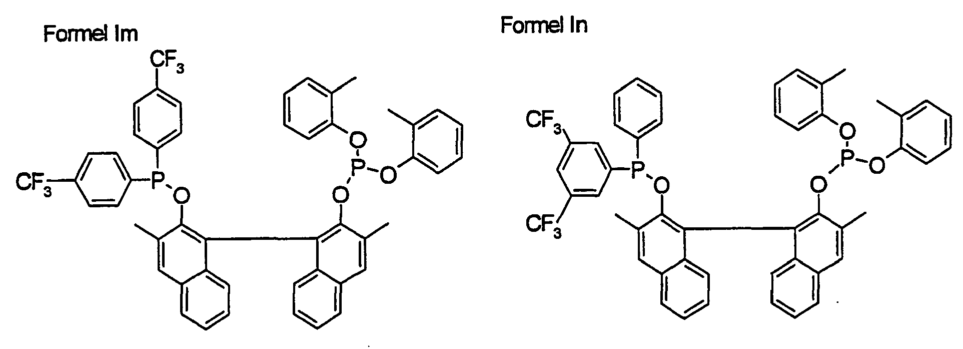

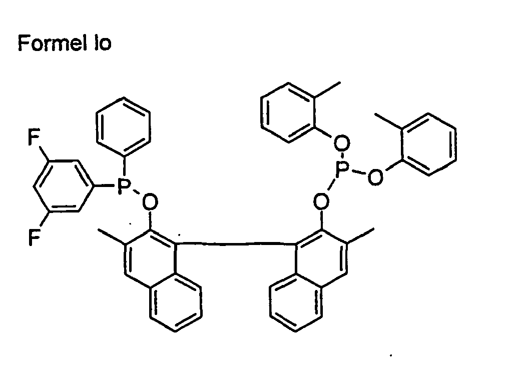

In Tabelle 1 haben die Abkürzungen folgende Bedeutungen:
H: Wasserstoff
Me: Methyl
Et: Ethyl
n-Pr: n-Propyl
t-Bu: t-Butyl

Zur Herstellung von Phosphinitphosphit I kann entsprechend dem in den US Patenten Nr. 5,523,453 und 5,693,843, sowie in WO 03/62171 beschriebenen Herstellverfahren für die dort beschriebenen Phosphorchelatliganden beispielsweise durch Umsetzung eines gegebenenfalls substituierten (Xn-Phenyl)(Xn-Phenyl)phosphinchlorids mit einem die Gruppen R1, R2, R3 und R4, sowie R15 bis R22 tragenden Diol und anschließende Umsetzung mit einem (Rn-Phenoxy)(Rn-Phenoxy)phosphinchlorids erfolgen.

Die Darstellung gelingt effizient und ökonomisch aus leicht zugänglichen Edukten.

Die als Ausgangsverbindung eingesetzten Diphenylphosphinchloride und deren Herstellung ist an sich bekannt, beispielsweise aus: H. Schindlbauer, Monatshefte Chemie, Band 96, 1965, Seite 1936-1942. Das dort beschriebene Verfahren zur Herstellung von 4-Fluorphenyldichlorphosphin kann analog zur Herstellung der (Xn-Phenyl)(Xn-Phenyl)phosphinchloride angewandt werden. Die zur Herstellung der jeweiligen Phenyl)-(Xn-Phenyl)phosphinchloride optimalen Parameter können dabei leicht durch wenige einfache Vorversuche ermittelt werden.

Die Phosphinitphosphite I können als Liganden in Übergangsmetallkomplexen eingesetzt werden.

Als Übergangsmetalle kommen dabei vorteilhaft die Metalle der 1. bis 2. und 6. bis 8. Nebengruppe des Periodensystems, vorzugsweise der 8. Nebengruppe des Periodensystems, besonders bevorzugt Eisen, Kobalt und Nickel, insbesondere Nickel in Betracht.

Setzt man Nickel ein, so kann dieses in verschiedenen Wertigkeiten, wie 0, +1, +2, +3, vorliegen. Bevorzugt ist hierbei Nickel(0) und Nickel(+2), insbesondere Nickel(0).

Zur Herstellung der Übergangsmetallkomplexe kann man eine ein Übergangsmetall enthaltende chemische Verbindung oder vorzugsweise ein Übergangsmetall mit einem Phosphinitphosphit I umsetzen, wobei als Phosphinitphosphit I ein einzelnes Phosphinitphosphit I oder ein Gemisch mehrerer Phosphinitphosphite I eingesetzt werden kann.

Das Übergangsmetall kann vor der Umsetzung aus geeigneten chemischen Verbindungen, beispielsweise durch Reduktion mit unedlen Metallen wie Zink, aus Salzen, wie Chloriden, erhalten werden.

Setzt man zur Herstellung der Übergangsmetallkomplexe eine ein Übergangsmetall enthaltende Verbindung ein, so kommen hierzu vorteilhaft Salze, wie Chloride, Bromide, Acetylacetonate, Sulfate, Nitrate, beispielsweise Nickel(II)-chlorid, oder Ni(0)-Komplexverbindungen, wie Bis(1,5-cyclooctadien)Ni(0), in Betracht.

Nach der Umsetzung der ein Übergangsmetall enthaltenden Verbindung oder des Übergangsmetalls mit einem Phosphinitphosphit I kann die Wertigkeit des Übergangsmetalls in dem Komplex mit geeigneten Oxidations- oder Reduktionsmitteln, beispielsweise unedlen Metallen, wie Zink, oder Wasserstoff in chemisch gebundener Form, wie Natriumborhydrid, oder in molekularer Form, oder elektrochemisch verändert werden.

In einer besonders bevorzugten Ausführungsform kommt die Umsetzung einer Komplexverbindung von Ni(0) mit organischen Monophosphin-, Monophosphinit-, Monophosphonit- oder Monophosphit-Liganden mit einem Phosphinitphosphit I in Betracht entsprechend dem in der deutschen Anmeldung 10136488.1 beschriebenen Verfahren.

In den Übergangsmetallkomplexen kann das molare Verhältnis von Übergangsmetall zu Phosphinitphosphit I im Bereich zwischen 1 und 6, vorzugsweise 2 bis 5, insbesondere 2, 3 oder 4, betragen.

Die Übergangsmetallkomplexe können frei von anderen Liganden als den Phosphinitphosphiten I sein.

Die Übergangsmetallkomplexe können neben den Phosphinitphosphiten I weitere Liganden enthalten, beispielsweise Nitrile, wie Acetonitril, Adipodinitril, 3-Pentennitril, 4-Pentennitril, 2-Methyl-3-butennitril, Olefine, wie Butadien, oder PhosphorVerbindungen, wie organische Monophosphine, Monophosphinite, Monophosphonite oder Monophosphite.

Die Herstellung solcher Übergangsmetallkomplexe kann grundsätzlich in einer solchen Weise erfolgen, wie sie in der Literatur, beispielsweise in DE-OS-2 237 703, US-A-3,850,973, US-A-3,766,237 oder US-A-3,903,120, zur Herstellung von Übergangsmetallkomplexen, die Tri-o-tolyl-phosphit, Tri-m-tolyl-phosphit oder Tri-p-tolyl-phosphit enthalten, beschrieben ist, indem man diese Phosphite durch die erfindungsgemäßen Phosphinitphosphite I teilweise oder vollständig ersetzt.

Die erfindungsgemäßen Übergangsmetallkomplexe können als Katalysator, insbesondere als Homogenkatalysator, eingesetzt werden.

Als besonders vorteilhaft hat sich die Verwendung der erfindungsgemäßen Übergangsmetallkomplexe als Katalysator bei der Addition von Blausäure an olefinische Doppelbindungen, insbesondere solche, die in Konjugation zu einer weiteren olefinischen Doppelbindung stehen, beispielsweise von Butadien unter Erhalt einer Mischung, enthaltend 2-Methyl-3-butennitril und 3-Pentennitril, erwiesen. Gleichermaßen vorteilhaft ist auch die Verwendung als Katalysator bei der Addition von Blausäure an olefinische Doppelbindungen, die nicht in Verbindung mit einer weiteren olefinischen Doppelbindung stehen, beispielsweise von 3-Pentennitril oder 4-Pentennitril oder deren Gemische, vorzugsweise 3-Pentennitril, unter Erhalt von Adipodinitril, oder von 3-Pentensäureester oder 4-Pentensäureester oder deren Gemische, vorzugsweise 3-Pentensäureester, unter Erhalt von 5-Cyanovaleriansäureester.

Ebenso als besonders vorteilhaft hat sich die Verwendung der erfindungsgemäßen Übergangsmetallkomplexe als Katalysator bei der Isomerisierung organischer Nitrile, insbesondere solcher, bei denen die Nitrilgruppe nicht in Konjugation zu einer olefinischen Doppelbindung steht, beispielsweise von 2-Methyl-3-butennitrit unter Erhalt von 3-Pentennitril, erwiesen. Gleichermaßen vorteilhaft ist auch die Verwendung als Katalysator bei der Isomerisierung organischer Nitrile, bei denen die Nitrilgruppe in Konjugation zu einer olefinischen Doppelbindung steht.

Verfahren zur Addition von Blausäure an eine olefinische Doppelbindung oder zur Isomerisierung von organischen Nitrilen kann grundsätzlich in einer solchen Weise erfolgen, wie sie beispielsweise in WO 99/13983 oder WO 99/64155 beschrieben ist, indem man die dort beschriebenen Phosphonite durch die erfindungsgemäßen Phosphinitphosphite I teilweise oder vollständig ersetzt.

Ein weiterer Gegenstand der Erfindung ist ein Verfahren zur Herstellung von Gemischen monoolefinischer C₅-Mononitrile mit nichtkonjugierter C=C- und C=N-Bindung durch Hydrocyanierung eines 1,3-Butadien-haltigen Kohlenwasserstoffgemisches in Gegenwart eines Katalysators, das dadurch gekennzeichnet ist, dass die Hydrocyanierung in Gegenwart mindestens eines der zuvor beschriebenen erfindungsgemäßen Systeme erfolgt.

Vorzugsweise wird zur Herstellung von monoolefinischen C₅-Mononitrilen nach dem erfindungsgemäßen Verfahren ein Kohlenwasserstoffgemisch eingesetzt, das einen 1,3-Butadien-Gehalt von mindestens 10 Vol.-%, bevorzugt mindestens 25 Vol.-%, insbesondere mindestens 40 Vol.-%, aufweist.

Zur Herstellung von Gemischen monoolefinischer C₅-Mononitrile, die z.B. 3-Pentennitril und 2-Methyl-3-butennitril enthalten und die als Zwischenprodukte für die Weiterverarbeitung zur Adipodinitril geeignet sind, kann man reines Butadien oder 1,3-Butadien-haltige Kohlenwasserstoffgemische einsetzen.

1,3-Butadien-haltige Kohlenwasserstoffgemische sind in großtechnischem Maßstab erhältlich. So fällt z.B. bei der Aufarbeitung von Erdöl durch Steamcracken von Naphtha ein als C₄-Schnitt bezeichnetes Kohlenwasserstoffgemisch mit einem hohen Gesamtolefinanteil an, wobei etwa 40 % auf 1,3-Butadien und der Rest auf Monoolefine und mehrfach ungesättigte Kohlenwasserstoffe sowie Alkane entfällt. Diese Ströme enthalten immer auch geringe Anteile von im Allgemeinen bis zu 5 % an Alkinen, 1,2-Dienen und Vinylacetylen.

Reines 1,3-Butadien kann z. B. durch extraktive Destillation aus technisch erhältlichen Kohlenwasserstoffgemischen isoliert werden.

C₄-Schnitte werden gegebenenfalls von Alkinen, wie z. B. Propin oder Butin, von 1,2-Dienen, wie z. B. Propadien, und von Alkeninen, wie z. B. Vinylacetylen, im wesentlichen befreit. Ansonsten werden unter Umständen Produkte erhalten, bei denen eine C=C-Doppelbindung in Konjugation mit der C≡N-Bindung steht. Aus "Applied Homogeneous Catalysis with Organometalic Compounds", Bd. 1, VCH Weinheim, S. 479 ist bekannt, dass das bei der Isomerisierung von 2-Methyl-3-butennitril und 3-Pentennitril entstehende, konjugierte 2-Pentennitril als ein Reaktionsinhibitor für die Zweitaddition von Cyanwasserstoff zu Adipinsäuredinitril wirkt. Es wurde festgestellt, dass die oben genannten, bei der Hydrocyanierung eines nicht vorbehandelten C₄-Schnittes erhaltenen konjugierten Nitrile auch als Katalysatorgifte für den ersten Reaktionsschritt der Adipinsäureherstellung, die Monoaddition von Cyanwasserstoff, wirken.

Daher entfernt man gegebenenfalls aus dem Kohlenwasserstoffgemisch solche Komponenten teilweise oder vollständig, die bei katalytischer Hydrocyanierung Katalysatorgifte ergeben, insbesondere Alkine, 1,2-Diene und Gemische davon. Zur Entfernung dieser Komponenten wird der C₄-Schnitt vor der Addition von Cyanwasserstoff vorzugsweise einer katalytischen Teilhydrierung unterzogen. Diese Teilhydrierung erfolgt in Gegenwart eines Hydrierungskatalysators, der im Weesentlichen befähigt ist, Alkine und 1,2-Diene selektiv neben anderen Dienen und Monoolefinen zu hydrieren.

Geeignete heterogene Katalysatorsysteme umfassen im Allgemeinen eine Übergangsmetallverbindung auf einem inerten Träger. Geeignete anorganische Träger sind die hierfür üblichen Oxide, insbesondere Silicium- und Aluminiumoxide, Alumosilikate, Zeolithe, Carbide, Nitride etc. und deren Mischungen. Bevorzugt werden als Träger Al₂O₃, SiO₂ und deren Mischungen verwendet. Insbesondere handelt es sich bei den verwendeten heterogenen Katalysatoren um die in den US-A-4,587,369; US-A-4,704,492 und US-A-4,493,906 beschriebenen, auf die hier in vollem Umfang Bezug genommen wird. Weiterhin geeignete Katalysatorsysteme auf Cu-Basis werden von der Fa. Dow Chemical als KLP-Katalysator vertrieben.

Die Addition von Cyanwasserstoff an 1,3-Butadien oder ein 1,3-Butadien-haltiges Kohlenwasserstoffgemisch, z. B. einen vorbehandelten, teilhydrierten C₄-Schnitt, kann kontinuierlich, semikontinuierlich oder diskontinuierlich erfolgen.

Nach einer geeigneten Variante des erfindungsgemäßen Verfahrens erfolgt die Addition des Cyanwasserstoffs kontinuierlich. Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben. Vorzugsweise wird für die kontinuierliche Variante des erfindungsgemäßen Verfahrens eine Rührkesselkaskade oder ein Rohrreaktor verwendet.

Gemäß einer bevorzugten Variante des erfindungsgemäßen Verfahrens erfolgt die Addition des Cyanwasserstoffs an 1,3-Butadien oder ein 1,3-Butadien-haltiges Kohlenwasserstoffgemisch semikontinuierlich.

Das semikontinuierliche Verfahren umfasst:
a) Befüllen eines Reaktors mit dem Kohlenwasserstoffgemisch, gegebenenfalls einem Teil des Cyanwasserstoffs und einem gegebenenfalls in situ erzeugten, erfindungsgemäßen Hydrocyanierungskatalysator sowie gegebenenfalls einem Lösungsmittel,
b) Umsetzung des Gemisches bei erhöhter Temperatur und erhöhtem Druck, wobei bei semikontinuierlicher Fahrweise Cyanwasserstoff nach Maßgabe seines Verbrauchs eingespeist wird,
c) Vervollständigung des Umsatzes durch Nachreagieren und anschließende Aufarbeitung.

Geeignete druckfeste Reaktoren sind dem Fachmann bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann. Für die obigen Schritte gilt vorzugsweise folgendes zu beachten:

### Schritt a):

Der druckfeste Reaktor wird vor Beginn der Reaktion mit dem teilhydrierten C₄-Schnitt oder Butadien, Cyanwasserstoff einem Hydrocyanierungskatalysator sowie ggf. einem Lösungsmittel befüllt. Geeignete Lösungsmittel sind dabei die zuvor bei der Herstellung der erfindungsgemäßen Katalysatoren genannten, bevorzugt aromatischen Kohlenwasserstoffe, wie Toluol und Xylol, oder Tetrahydrofuran.

### Schritt b):

Die Umsetzung des Gemisches erfolgt im Allgemeinen bei erhöhter Temperatur und erhöhtem Druck. Dabei liegt die Reaktionstemperatur im Allgemeinen in einem Bereich von etwa 0 bis 200°C, bevorzugt etwa 50 bis 150°C. Der Druck liegt im Allgemeinen in einem Bereich von etwa 1 bis 200 bar, bevorzugt etwa 1 bis 100 bar, insbesondere 1 bis 50 bar, insbesondere bevorzugt 1 bis 20 bar. Dabei wird während der Reaktion Cyanwasserstoff nach Maßgabe seines Verbrauchs eingespeist, wobei der Druck im Autoklaven im Wesentlichen konstant bleibt. Die Reaktionszeit beträgt etwa 30 Minuten bis 5 Stunden.

### Schritt c):

Zur Vervollständigung des Umsatzes kann sich an die Reaktionszeit eine Nachreaktionszeit von 0 Minuten bis etwa 5 Stunden, bevorzugt etwa 1 Stunde bis 3,5 Stunden anschließen, in der kein Cyanwasserstoff mehr in den Autoklaven eingespeist wird. Die Temperatur wird in dieser Zeit im Wesentlichen konstant auf der zuvor eingestellten Reaktionstemperatur belassen. Die Aufarbeitung erfolgt nach gängigen Verfahren und umfaßt die Abtrennung des nicht umgesetzten 1,3-Butadiens und des nicht umgesetzten Cyanwasserstoffs, z. B. durch Waschen oder Extrahieren und die destillative Aufarbeitung des übrigen Reaktionsgemisches zur Abtrennung der Wertprodukte und Rückgewinnung des noch aktiven Katalysators.

Gemäß einer weiteren geeigneten Variante des erfindungsgemäßen Verfahrens erfolgt die Addition des Cyanwasserstoffs an das 1,3-Butadien-haltige Kohlenwasserstoffgemisch diskontinuierlich. Dabei werden im Wesentlichen die bei semikontinuierlichen Verfahren beschriebenen Reaktionsbedingungen eingehalten, wobei in Schritt b) kein zusätzlicher Cyanwasserstoff eingespeist, sondern dieser komplett vorgelegt wird.

Allgemein läßt sich die Herstellung von Adipinsäuredinitril aus einem Butadien-haltigen Gemisch durch Addition von 2 Moläquivalenten Cyanwasserstoff in drei Schritte gliedern:
1. Herstellung von C₅-Monoolefingemischen mit Nitrilfunktion.
2. Isomerisierung des in diesen Gemischen enthaltenen 2-Methyl-3-butennitrils zu 3-Pentennitril und Isomerisierung des so gebildeten und des in den Gemischen bereits aus Schritt 1 enthaltenen 3-Pentennitrils zu verschiedenen n-Pentennitrilen. Dabei soll ein möglichst hoher Anteil an 3-Pentennitril bzw. 4-Pentennitril und ein möglichst geringer Anteil an konjugiertem und gegebenenfalls als Katalysatorgift wirksamen 2-Pentennitril und 2-Methyl-2-butennitril gebildet werden.
3. Herstellung von Adipinsäuredinitril durch Addition von Cyanwasserstoff an das in Schritt 2 gebildete 3-Pentennitril welches zuvor "in situ" zu 4-Pentennitril isomerisiert wird. Als Nebenprodukte treten dabei z. B. 2-Methyl-glutarodinitril aus der Markownikow-Addition von Cyanwasserstoff an 4-Pentennitril oder der anti-Markownikow-Addition von Cyanwasserstoff an 3-Pentennitril und Ethylsuccinodinitril aus der Markownikow-Addition von Cyanwasserstoff an 3-Pentennitril auf.

Vorteilhafterweise eignen sich die erfindungsgemäßen Katalysatoren auf Basis von Phosphinitliganden auch für die Stellungs- und Doppelbindungsisomerisierung in Schritt 2 und/oder die Zweitaddition von Cyanwasserstoff in Schritt 3.

Vorteilhafterweise zeigen die erfindungsgemäß eingesetzten Katalysatoren nicht nur eine hohe Selektivität im Bezug auf die bei der Hydrocyanierung von 1,3-Butadien-haltigen Kohlenwasserstoffgemischen erhaltenen Monoadditionsprodukte, sondern sie können bei der Hydrocyanierung auch mit einem Überschuss an Cyanwasserstoff versetzt werden, ohne dass es zu einer merklichen Abscheidung von inaktiven Nickel(II)-Verbindungen, wie z.B. Nickel(II)-cyanid, kommt. Im Gegensatz zu bekannten Hydrocyanierungskatalysatoren auf Basis nicht-komplexer Phosphin- und Phosphitliganden eignen sich die Katalysatoren, enthaltend ein Phosphinitphosphit 1, somit nicht nur für kontinuierliche Hydrocyanierungsverfahren, bei denen ein Cyanwasserstoffüberschuss im Reaktionsgemisch im Allgemeinen wirkungsvoll vermieden werden kann, sondern auch für semikontinuierliche Verfahren und Batch-Verfahren, bei denen im Allgemeinen ein starker Cyanwasserstoffüberschuss vorliegt. Somit weisen die erfindungsgemäß eingesetzten Katalysatoren und die auf ihnen basierenden Verfahren zur Hydrocyanierung im Allgemeinen höhere Katalysatorrückführungsraten und längere Katalysatorstandzeiten auf als bekannte Verfahren. Dies ist neben einer besseren Wirtschaftlichkeit auch unter ökologischen Aspekten vorteilhaft, da das aus dem aktiven Katalysator mit Cyanwasserstoff gebildete Nickelcyanid stark giftig ist und unter hohen Kosten aufgearbeitet oder entsorgt werden muss.

Neben der Hydrocyanierung von 1,3-Butadien-haltigen Kohlenwasserstoffgemischen eignen sich die erfindungsgemäßen Systeme im Allgemeinen für alle gängigen Hydrocyanierungsverfahren. Dabei sei insbesondere die Hydrocyanierung von nichtaktivierten Olefinen, z.B. von Styrol und 3-Pentennitril, genannt.

Die Addition von Blausäure an eine olefinische Doppelbindung in Gegenwart eines erfindungsgemäßen Katalysatorsystems, insbesondere die Addition an Butadien, ein Butadien oder an 3-Pentennitril, 4-Pentennitril oder Gemische solcher Pentennitrile, oder die Isomerisierug organischer Nitrile in Gegenwart eines erfindungsgemäßen Katalysatorsystems, insbesondere die Isomerisierung von 2-Methyl-3-butennitril zu 3-Pentennitril, kann vorteilhaft in Gegenwart von einer oder mehreren Lewis-Säuren als Promotoren durchgeführt werden, die Aktivität, Selektivität oder beides des erfindungsgemäßen Katalysatorsystems beeinflussen. Als Promotoren kommen anorganische und organische Verbindungen in Betracht, in denen das Kation ausgewählt ist aus der Gruppe bestehend aus Scandium, Titan, Vanadium, Chrom, Mangan, Eisen, Cobalt, Kupfer, Zink, Bor, Aluminium, Yttrium, Zirkonium, Niob, Molybdän, Cadmium, Rhenium und Zinn. Beispielhaft seien genannt ZnBr₂, Znl₂, ZnCl₂, ZnSO₄, CuCl₂, CuCl, Cu(OₛSCF₃)₂, CoCl₂, Col₂, Fel₂, FeCl₃, FeCl₂, FeCl₂(THF)₂, TiCl₄(THF)₂, TiCl₄, TiCl₃, ClTi(O-iso-Pr)₃, MnCl₂, ScCl₃, AlCl₃, (C₂H₅)AlCl₂, (C₂H₅)₂AlCl, (C₂H₅)₃Al₂Cl₃, (C₈H₁₇)AlCl₂, (C₈H₁₇)₂AlCl, (iso-C₄H₉)₂AlCl, Ph₂AlCl, PhAlCl₂, ReCl₅, ArCl₄, ZrCl₂, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆Hₛ)₃, TaCl₅, wie sie allgemein in US 6,171,996 B1 beschrieben sind. Geeignete Promotoren sind weiterhin beschrieben in US 3,496,217, US 3,496,218 und US 4,774,353. Diese umfassen Metallsalze, wie ZnCl₂, Col₂ und SnCl₂, und organometallische Verbindungen, wie RAlCl₂, R₃SnO₃SCF₃, und R₃B, wobei R eine Alkylgruppe oder Arylgruppe ist. US 4,874,884 beschreibt, wie synergistisch wirksame Kombinationen von Promotoren ausgewählt werden können, um die katalytische Aktivität des Katalysatorsystems zu erhöhen. Bevorzugte Promotoren umfassen CdCl₂, FeCl₂, ZnCl₂, B(C₆H₅)₃ und (C₆H₅)₃SnZ, wobei Z für CF₃SO₃, CH₃C₆H₄SO₃ oder(C₆H₅)₃BCN steht.

Das molare Verhältnis von Promotor zu Nickel in dem Katalysatorsystem kann zwischen 1:16 bis 50:1 liegen.

Eine weitere vorteilhafte Ausführungsform der Hydrocyanierung und Isomerisierung kann US 5,981,772 entnommen werden, deren Inhalt durch Bezugnahme hiermit in die vorliegende Erfindung eingeschlossen wird, mit der Maßgabe, das anstelle der in dieser Patentschrift genannten Katalysatoren ein erfindungsgemäßes System oder ein Gemisch solcher Systeme eingesetzt wird.

Eine weitere vorteilhafte Ausführungsform der Hydrocyanierung und Isomerisierung kann US 6,127,567 entnommen werden, deren Inhalt durch Bezugnahme hiermit in die vorliegende Erfindung eingeschlossen wird, mit der Maßgabe, das anstelle der in dieser Patentschrift genannten Katalysatoren ein erfindungsgemäßes System oder ein Gemisch solcher Systeme eingesetzt wird.

Eine weitere vorteilhafte Ausführungsform der Hydrocyanierung kann US 5,693,843 entnommen werden, deren Inhalt durch Bezugnahme hiermit in die vorliegende Erfindung eingeschlossen wird, mit der Maßgabe, das anstelle der in dieser Patentschrift genannten Katalysatoren ein erfindungsgemäßes System oder ein Gemisch solcher Systeme eingesetzt wird.

Eine weitere vorteilhafte Ausführungsform der Hydrocyanierung kann US 5,523,453 entnommen werden, deren Inhalt durch Bezugnahme hiermit in die vorliegende Erfindung eingeschlossen wird, mit der Maßgabe, das anstelle der in dieser Patentschrift genannten Katalysatoren ein erfindungsgemäßes System oder ein Gemisch solcher Systeme eingesetzt wird.

Die Erfindung wird anhand der folgenden, nicht einschränkenden Beispiele näher erläutert.

### Ausführungsbeispiele

Die Ausbeuten wurden gaschromatographisch bestimmt (Säule: 30 m HP-50, Temperaturprogramm: 11 Minuten isotherm bei 40°C, danach Aufheizen mit einer Geschwindigkeit von 10 °C/min auf 280°C, Gaschromatographie: Hewlett Packard HP 5890)

Sämtliche Beispiele wurden unter einer Schutzatmosphäre aus Argon durchgeführt. Die vorteilhafte Spezifikation der Startmaterialen BD, HCN, 3PN und 2M3BN kann WO 03/045552 entnommen werden.

Die Abkürzung Nickel(O)-(m/p-Tolylphosphit) steht für eine Mischung enthaltend 2,35 Gew.-% Ni(0), 19 Gew.-% 3-Pentennitril und 78,65 Gew.-% m/p-Tolylphosphit mit einem m:p-Verhältnis von 2:1.

Als Chelatliganden wurden eingesetzt:

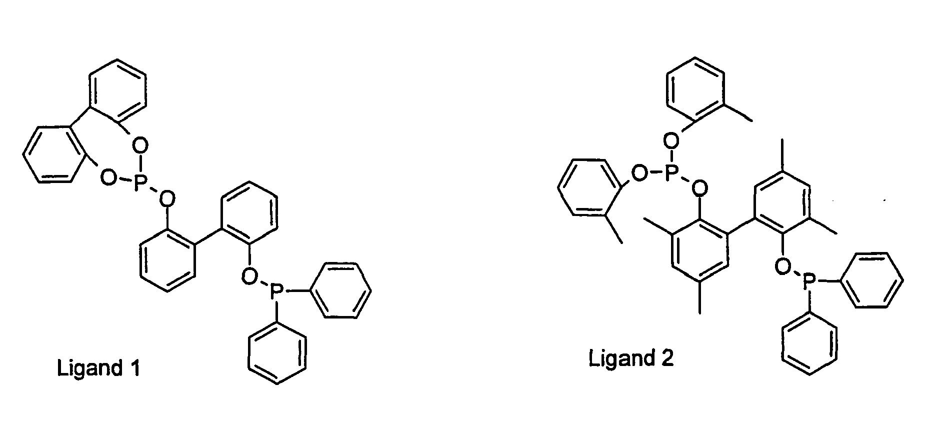

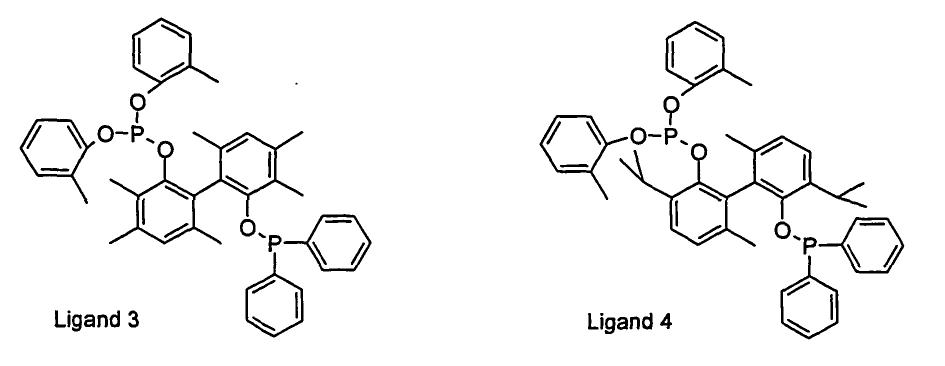

Ni(COD)₂ steht für Ni(O)-bis-(1,4-cyclooctadien), 2M3BN für 2-Methyl-3-butennitril, t2M2BN für trans-2-Methyl-2-butennitril, c2M2BN für cis-2-Methyl-2-butennitril, t2PN für trans-2-Pentennitril, 4PN für 4-Pentennitril, t3PN für trans-3-Pentennitril, c3PN für cis-3-Pentennitril, MGN für Methylglutaronitril, 3PN für die Summe aus t3PN und c3PN, BD für 1,3-Butadien, HCN für Blausäure, ADN für Adipodinitril und THF für Tetrahydrofuran.

### Beispiel 1-3: Hydrocyanierung von BD zu 2M3BN/3PN mit anschließender 2M3BN-Isomerisierung

### Beispiel 1 (vergleich): (0,51 mmol Ni(O))

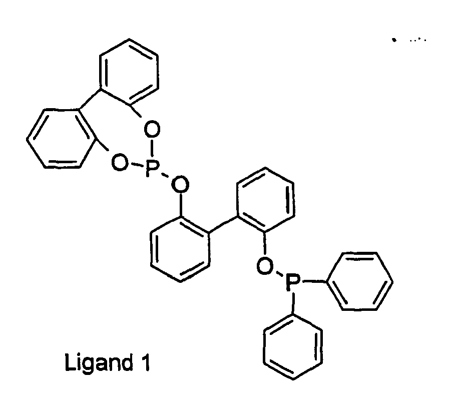

1 Äq.-Ni(COD)₂ wird mit 3 Äq. Ligand 1 in THF 20 Min. gerührt. Diese Lösung wird mit 797 Äq. BD versetzt, bei 25°C in einen Glasautoklaven gefüllt und auf 90°C erwärmt. Über 60 Min. werden nun 465 Äq. HCN in THF zudosiert und weitere 75 Min. bei 90°C nachgerührt. Man bestimmt nach 135 Min das Verhältnis 2M3BN/3PN GC-Chromatographisch (GC-Flächenprozent). Das Verhältnis 2M3BN/3PN betrug 1,9/1.

Anschließend wird der Gesamte Ansatz auf 115°C für 60 Min, erwärmt, um 2M3BN direkt zu 3PN zu isomerisieren.

Der HCN-Umsatz zu 2M3BN/3PN betrug > 95% (GC-Gewichtsprozent, interner Standart: Ethylbenzol). Das Verhältnis 2M3BN/3PN betrug 1,8/1.

### Beispiel 2 (erfindungsgemäß): (0,53 mmol Ni(O))

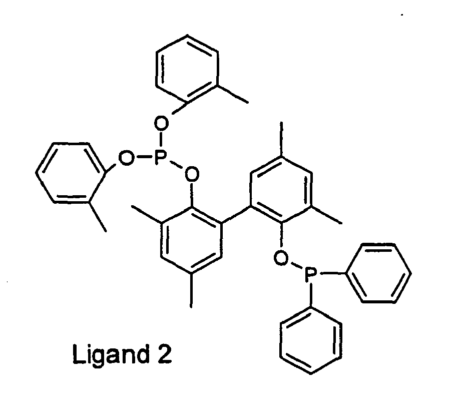

### Ligandsynthese:

In einer Argonatmosphäre werden in einen 500ml Kolben 40 mmol 2,2'-Dihydroxy-3,3',5,5'-Tetramethylbiphenol und 160 mmol Triethylamin bei -15°C in 120 ml Toluol vorgelegt. Bei dieser Temperatur werden 44 mmol Diphenylchlorphosphin gelöst in 40 ml Toluol innerhalb von 40 Min. zugetropft. Die Mischung wird 6h bei -15°C nachgerührt. Bei -15°C wird in die Mischung 40 mmol Di-o-Kresylchlorphosphit, gelöst in 40 ml Toluol, getropft. Man lässt auf Raumtemperator kommen und rührt 15h nach. Der Ansatz wird filtriert und das Filtrat vollständig eingeengt. Man erhält 25,3g Produkt. ³¹P-NMR (C₆D₆): 133,5 ppm und 112,8 ppm; Bisphosphinitverunreinigungen 112,5 ppm.

1 Äq.-Ni(COD)₂ wird mit 3 Äq. Ligand 2 in THF 20 Min. gerührt. Diese Lösung wird mit 740 Äq. BD versetzt, bei 25°C in einen Glasautoklaven gefüllt und auf 80°C erwärmt. Über 100 Min. werden nun 465 Äq. HCN in THF zudosiert und weitere 20 Min. bei 80°C nachgerührt. Man bestimmt nach 120 Min das Verhältnis 2M3BN/3PN GC-Chromatographisch (GC-Flächenprozent. Das Verhältnis 2M3BN/3PNbetrug 1,5/1.

Anschließend wird der Gesamte Ansatz auf 115°C für 60 Min. erwärmt, um 2M3BN direkt zu 3PN zu isomerisieren.

Der HCN-Umsatz zu 2M3BN/3PN betrug > 95% (GC-Gewichtsprozent, interner Standart: Ethylbenzol). Das Verhältnis 2M3BN/3PN betrug 1/4,6.

### Beispiel 3 (erfindungsgemäß): (0,76 mmol Ni(0))

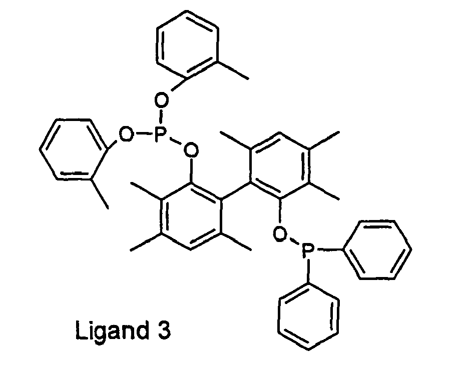

### Ligandsynthese:

In einer Argonatmosphäre werden in einen 500ml Kolben 40 mmol 2,2'-Dihydroxy-3,3',5,5',6,6'-Hexamethylbiphenol und 160 mmol Triethylamin bei -15°C in 120 ml Toluol vorgelegt. Bei dieser Temperatur werden 44 mmol Diphenylchlorphosphin gelöst in 40 ml Toluol innerhalb von 40 Min. zugetropft. Die Mischung wird 6h bei -15°C nachgerührt. Bei -15°C wird in die Mischung 40 mmol Di-o-Kresylchlorphosphit, gelöst in 40 ml Toluol, getropft. Man lässt auf Raumtemperator kommen und rührt 15h nach. Der Ansatz wird filtriert und das Filtrat vollständig eingeengt. Man erhält 21,5g Produkt. ³¹P-NMR (C₆D₆): 134,7 ppm und 110,6 ppm.

1 Äq.-Ni(COD)₂ wird mit 3 Äq. Ligand 3 in THF 20 Min. gerührt. Diese Lösung wird mit 770 Äq. BD versetzt, bei 25°C in einen Glasautoklaven gefüllt und auf 80°C erwärmt. Über 60 Min. werden nun 465 Äq. HCN in THF zudosiert und weitere 40 Min. bei 80°C nachgerührt. Man bestimmt nach 100 Min das Verhältnis 2M3BN/3PN GC-Chromatographisch (GC-Flächenprozent. Das Verhältnis 2M3BN/3PN betrug 2,5/1.

Anschließend wird der Gesamte Ansatz auf 115°C für 60 Min. erwärmt, um 2M3BN direkt zu 3PN zu isomerisieren.

Der HCN-Umsatz zu 2M3BN/3PN betrug > 95% (GC-Gewichtsprozent, interner Standart: Ethylbenzol). Das Verhältnis 2M3BN/3PN betrug 1/2,6.

### Beispiel 4-8: Isomerisierung von 2M3BN zu 3PN

### Beispiel 4 (vergleich): (0,5 mmol Ni(0))

1 Äq.Nickel(0)-(m.lp-Tolylphosphit)₅₋₇ wird mit 465 Äq. 2M3BN versetzt und auf 115°C erwärmt. Nach 90 Min. und nach 180 Min. werden GC-Proben aus dem Reaktionsgemisch entnommen und GC-Chromatographisch (GC-Flächenprozent) untersucht.

| Zeit | 2M3BN | t2M2BN | c2M2BN | t2PN | 4PN/t3PN/c3PN | 3PN/2M3BN |
|---|---|---|---|---|---|---|
| 90 Min | 84,5 | 1,3 | 0,3 | 0 | 13,0 | 0,15 |
| 180 Min | 72,4 | 1,5 | 0,5 | 0 | 24,4 | 0,34 |

### Beispiel 5 (vergleich): (0,51 mmol Ni(0))

1 Äq.-Ni(COD)₂ wird mit 3 Äq. Ligand 1 und 465 Äq. 2M3BN versetzt, 1 h bei 25°C gerührt und auf 115°C erwärmt. Nach 0, 1h und nach 3 h werden GC-Proben aus dem Reaktionsgemisch entnommen und GC-Chromatographisch (GC-Flächenprozent) untersucht.

| Zeit | 2M3BN | c,t-2M2BN | c,t-2PN | 4PN | c,t-3PN | 3PN/2M 3BN |
|---|---|---|---|---|---|---|
| 0h | 94,8 | 4,96 | 0 | 0 | 0 | 0 |
| 1 h | 86,04 | 5,98 | 0 | 0 | 6,85 | 0,08 |
| 3 h | 79,67 | 7,09 | 0 | 0 | 11,28 | 0,14 |

### Beispiel 6 (erfindungsgemäß): (0,4 mmol Ni(0))

1 Äq.-Ni(COD)₂ wird mit 3 Äq. Ligand 2 und 465 Äq. 2M3BN versetzt, 1 h bei 25°C gerührt und auf 115°C erwärmt. Nach 0, 5 Min. und nach 25 Min. werden GC-Proben aus dem Reaktionsgemisch entnommen und GC-Chromatographisch (GC-Flächenprozent) untersucht.

| Zeit | 2M3BN | 2M2BN | 2PN | 3PN + 4PN | 3PN/2M 3BN |
|---|---|---|---|---|---|
| 0Min | 85,5 | 4,1 | 0 | 8,4 | 0,1 |
| 5 Min | 51,4 | 4,1 | 0 | 42,3 | 1,2 |
| 25 Min | 4,8 | 4,0 | 0 | 89,1 | 18,6 |

### Beispiel 7 (erfindungsgemäß): (0,38 mmol Ni(0))

1 Äq.-Ni(COD)₂ wird mit 3 Äq. Ligand 3 und 465 Äq. 2M3BN versetzt, 1 h bei 25°C gerührt und auf 115°C erwärmt. Nach 0, 5 Min. und nach 25 Min. werden GC-Proben aus dem Reaktionsgemisch entnommen und GC-Chromatographisch (GC-Flächenprozent) untersucht.

| Zeit | 2M3BN | 2M2BN | 2PN | 3PN + 4PN | 3PN/2M 3BN |
|---|---|---|---|---|---|
| 0Min | 91,1 | 4,5 | 0 | 2,9 | 0,03 |
| 5 Min | 68,1 | 4,4 | 0 | 25,3 | 0,38 |
| 25 Min | 4,8 | 4,4 | 0,1 | 88,4 | 18,4 |

### Beispiel 8 (erfindungsgemäß): (0,35 mmol Ni(0))

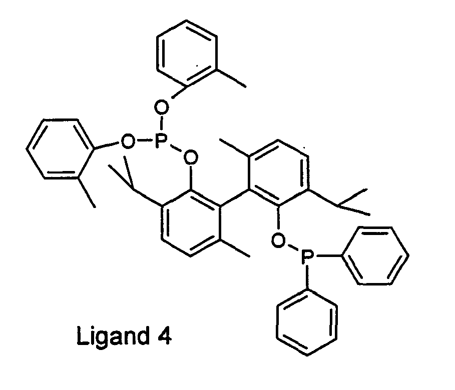

### Ligandsynthese:

In einer Argonatmosphäre werden in einen 500ml Kolben 40 mmol 2,2'-Dihydroxy-3,3'-Diisopropyl-6,6'-dimethylbiphenol und 44 mmol Diphenylchlorphosphin bei -15°C in 120 ml Toluol vorgelegt. Bei dieser Temperatur werden 160 mmol Triethylamin gelöst in 40 ml Toluol innerhalb von 40 Min. zugetropft. Die Mischung wird 6h bei -15°C nachgerührt. Bei -15°C wird in die Mischung 40 mmol Di-o-Kresylchlorphosphit, gelöst in 40 ml Toluol, getropft. Man lässt auf Raumtemperator kommen und rührt 15h nach. Der Ansatz wird filtriert und das Filtrat vollständig eingeengt. Man erhält 21,5g Produkt. ³¹P-NMR (C₆D₆): 132,5 ppm und 113,5 ppm.

1 Äq.-Ni(COD)₂ wird mit 3 Äq. Ligand 4 und 465 Äq. 2M3BN versetzt, 1 h bei 25°C gerührt und auf 115°C erwärmt. Nach 0, 5 Min. und nach 25 Min. werden GC-Proben aus dem Reaktionsgemisch entnommen und GC-Chromatographisch (GC-Flächenprozent) untersucht.

| Zeit | 2M3BN | 2M2BN | 2PN | 3PN + 4PN | 3PN/2M 3BN |
|---|---|---|---|---|---|
| 0Min | 89,1 | 4,8 | 0 | 4,2 | 0,05 |
| 5 Min | 78,8 | 4,6 | 0 | 14,9 | 0,19 |
| 25 Min | 41,5 | 4,4 | 0 | 52,5 | 1,27 |

### Beispiel 9-13: Hydrocyanierung von 3PN zu ADN

### Beispiel 9 (vergleich): (0,6 mmol Ni(0))

1 Äq.-Nickel(0)-(m-/p-Tolylphosphit)₅₋₇ wird mit 365 Äq. 3PN versetzt, eine Stunde bei 25°C gerührt und auf 70°C erwärmt. Zu dieser Mischung wird 1 Äq. ZnCl₂ zugegeben und weitere 5 Min. gerührt. In einem Ar-Trägergasstrom werden nun 94 Äq. HCN/h*Ni eingegast. Nach 30 Min., 60 Min. und 150 Min. werden GC-Proben aus dem Reaktionsgemisch entnommen und GC-Chromatographisch (GC-Gewichtsprozent, interner Standart: Ethylbenzol) untersucht.

| Zeit | MGN | ADN | ADN-Selektivität (%) |
|---|---|---|---|
| 30 Min | 3,35 | 10,75 | 76,2 |
| 60 Min | 6,87 | 26,39 | 79,3 |
| 150 Min | 7,11 | 27,82 | 79,6 |

### Beispiel 10 (Vergleich): (0,45 mmol Ni(0))

1 Äq.-Ni(COD)₂ wird mit 3 Äq. Ligand 1 und 365 Äq. 3PN versetzt, eine Stunde bei 25°C gerührt und auf 70°C erwärmt. Zu dieser Mischung wird 1 Äq. ZnCl₂ zugegeben und weitere 5 Min. gerührt. In einem Ar-Trägergasstrom werden nun 286 Äq. HCN/h*Ni eingegast. Nach 60 Min. wird eine GC-Probe aus dem Reaktionsgemisch entnommen und GC-Chromatographisch (GC-Gewichtsprozent, interner Standart: Ethylbenzol) untersucht.

| Zeit | MGN | ADN | ADN-Selektivität (%) |
|---|---|---|---|
| 60 Min | 1,4 | 8,4 | 86,0 |

### Beispiel 11 (erfindungsgemäß): (0,37 mmol Ni(0))

1 Äq.-Ni(COD)₂ wird mit 3 Äq. Ligand 2 und 365 Äq. 3PN versetzt, eine Stunde bei 25°C gerührt und auf 40°C erwärmt. Zu dieser Mischung wird 1 Äq. ZnCl₂ zugegeben und weitere 5 Min. gerührt. In einem Ar-Trägergasstrom werden nun 309 Äq. HCNIh*Ni eingegast. Nach 88 Min. wird eine GC-Probe aus dem Reaktionsgemisch entnommen und GC-Chromatographisch (GC-Gewichtsprozent, interner Standart: Ethylbenzol) untersucht.

| Zeit | MGN | ADN | ADN-Selektivität (%) |
|---|---|---|---|
| 88 Min | 6,5 | 68,3 | 91,3 |

### Beispiel 12 (erfindungsgemäß): (0,36 mmol Ni(0))

1 Äq.-Ni(COD)₂ wird mit 3 Äq. Ligand 3 und 365 Äq. 3PN versetzt, eine Stunde bei 25°C gerührt und auf 40°C erwärmt. Zu dieser Mischung wird 1 Aq. ZnCl₂ zugegeben und weitere 5 Min. gerührt. In einem Ar-Trägergasstrom werden nun 302 Äq. HCN/h*Ni eingegast. Nach 80 Min. wird eine GC-Probe aus dem Reaktionsgemisch entnommen und GC-Chromatographisch (GC-Gewichtsprozent, interner Standart: Ethylbenzol) untersucht.

| Zeit | MGN | ADN | ADN-Selektivität (%) |
|---|---|---|---|
| 80 Min | 7,2 | 62,4 | 89,7 |

### Beispiel 13 (erfindungsgemäß): (0,39 mmol Ni(0))

1 Äq.-Ni(COD)₂ wird mit 3 Äq. Ligand 4 und 365 Äq. 3PN versetzt, eine Stunde bei 25°C gerührt und auf 40°C erwärmt. Zu dieser Mischung wird 1 Äq. ZnCl₂ zugegeben und weitere 5 Min. gerührt. In einem Ar-Trägergasstrom werden nun 289 Äq. HCN/h*Ni eingegast. Nach 82 Min. wird eine GC-Probe aus dem Reaktionsgemisch entnommen und GC-Chromatographisch (GC-Gewichtsprozent, interner Standart: Ethylbenzol) untersucht.

| Zeit | MGN | ADN | ADN-Selektivität (%) |
|---|---|---|---|
| 82 Min | 5,4 | 59,2 | 91,7 |

## Patentansprüche

1. Phosphinitphosphite I der Formel 1 oder 2 oder 3 oder 4 oder 5 oder 6 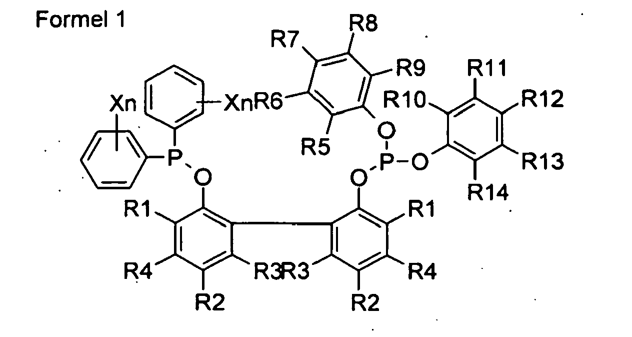 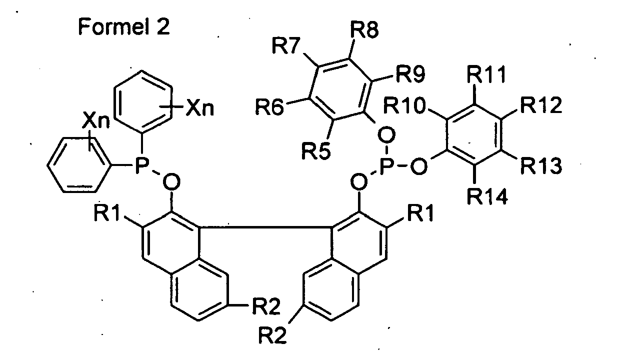 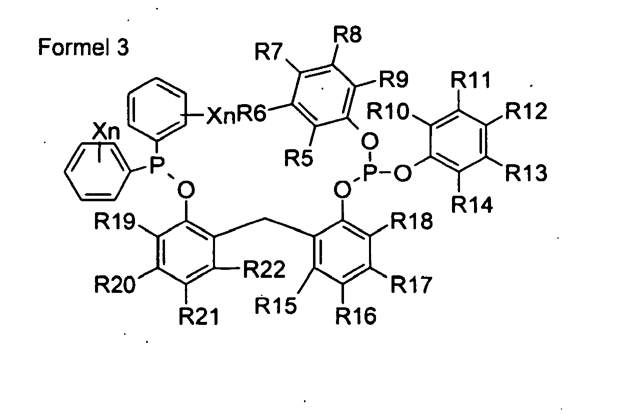 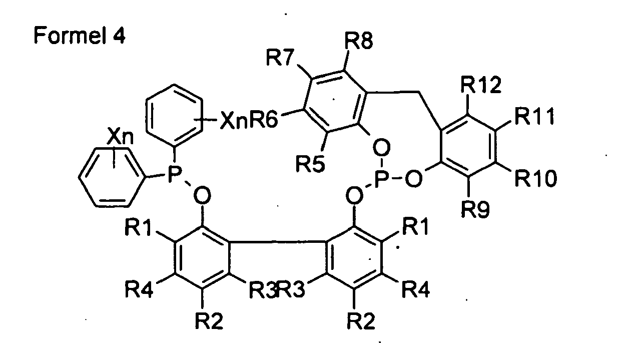 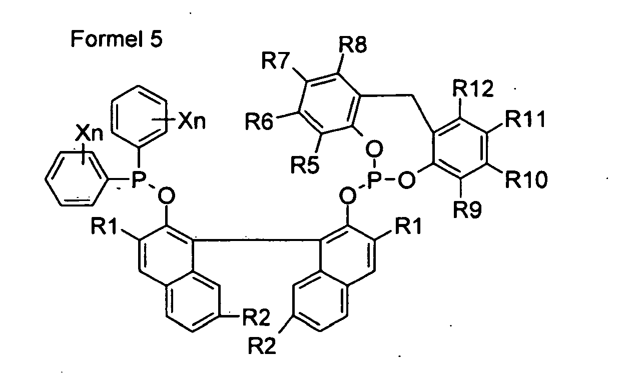 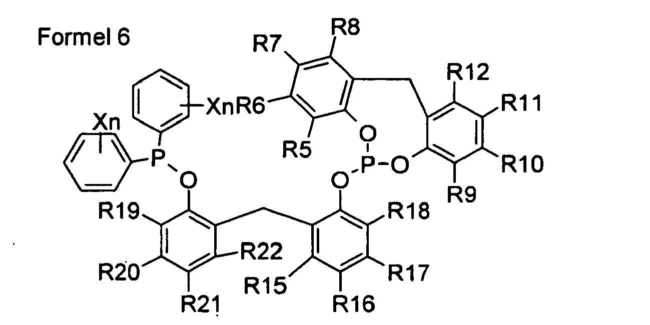 mit
R1, R2, R4 sind unabhängig voneinander H, eine Alkylgruppe mit 1 bis 8 Kohlenstoffatomen, mit der Maßgabe, dass mindestens eine der Gruppen R1, R2, R4 ungleich H ist,
R5 bis R22 sind unabhängig voneinander H, eine Alkylgruppe mit 1 bis 8 Kohlenstoffatomen,
R3 ist H, Methyl oder Ethyl,
X ist F, Cl oder CF₃, wenn n gleich 1 oder 2 ist,
X ist H, wenn n gleich 0 ist.
und deren Gemische.

2. Phosphinitphosphit I nach Anspruch 1 mit R1, R2, R4, R5, R7, R8, R10, R12, R13 unabhängig voneinander ausgewählt aus der Gruppe bestehend aus H, Methyl, Ethyl, n-Propyl, i-Propyl, und t-Butyl.

3. Verwendung eines Phosphinitphosphits I gemäß Anspruch 1 oder 2 als Ligand in Übergangsmetallkomplexen.

4. Übergangsmetallkomplexe enthaltend als Ligand ein Phosphinitphosphit I gemäß Anspruch 1 oder 2.

5. Übergangsmetallkomplexe nach Anspruch 4, wobei man als Übergangsmetall Nickel einsetzt.

6. Verfahren zur Herstellung von Übergangsmetallkomplexen gemäß Anspruch 4 oder 5, **dadurch gekennzeichnet, dass** man ein elementares Übergangsmetall oder eine ein Übergangsmetall enthaltende chemische Verbindung mit einem Phosphinitphosphit der Formel I gemäß Anspruch 1 oder 2 umsetzt.

7. Verwendung von Übergangsmetallkomplexen gemäß Anspruch 4 oder 5 als Katalysator.

8. Verwendung nach Anspruch 7 als Katalysator für die Addition von Blausäure an eine olefinische Doppelbindung.

9. Verwendung nach Anspruch 7 als Katalysator für die Isomerisierung organischer Nitrile.

10. Verfahren zur Addition von Blausäure an eine olefinische Doppelbindung in Gegenwart eines Katalysators, wobei man als Katalysator einen Übergangsmetallkomplex gemäß Anspruch 4 oder 5 einsetzt.

11. Verfahren nach Anspruch 10, wobei man Blausäure an Butadien addiert unter Erhalt einer Verbindung ausgewählt aus der Gruppe bestehend aus 2-Methyl-3-butennitril und 3-Pentennitril.

12. Verfahren nach Anspruch 10, wobei man Blausäure an ein 3-Pentennitril, 4-Pentennitril oder deren Gemische addiert unter Erhalt von Adipodinitril.

13. Verfahren zur Isomerisierung organischer Nitrile in Gegenwart eines Katalysators, wobei man als Katalysator einen Übergangsmetallkomplex gemäß Anspruch 4 oder 5 einsetzt.

14. Verfahren nach Anspruch 13, wobei man 2-Methyl-3-butennitril zu 3-Pentennitril isomerisiert.

## Claims

1. A phosphinite phosphite I of the formula 1 or 2 or 3 or 4 or 5 or 6 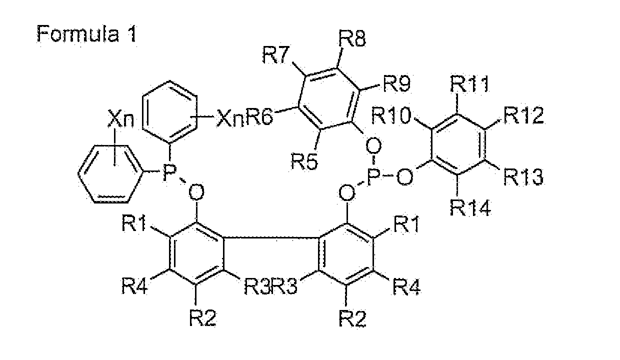 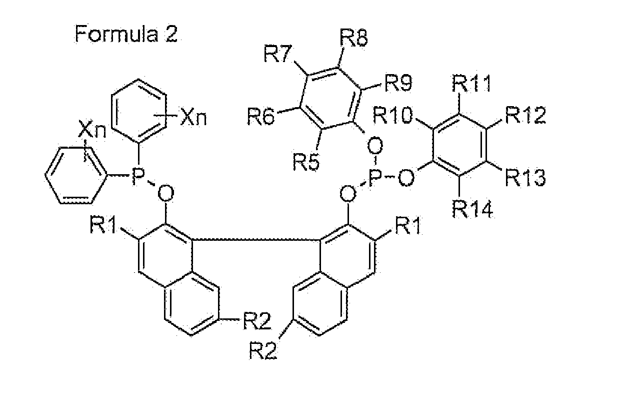 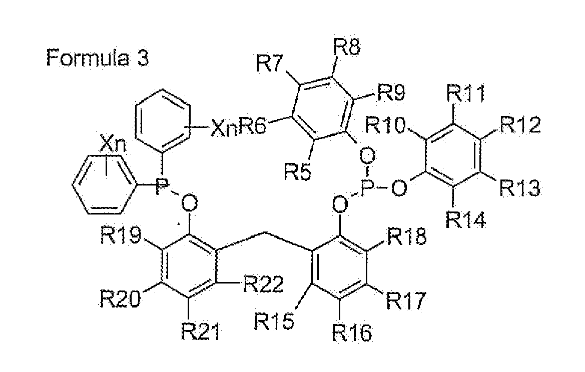 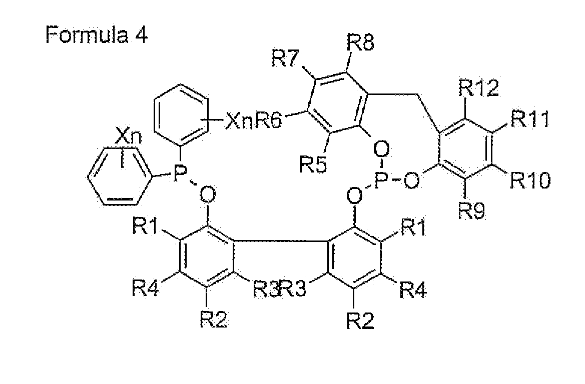 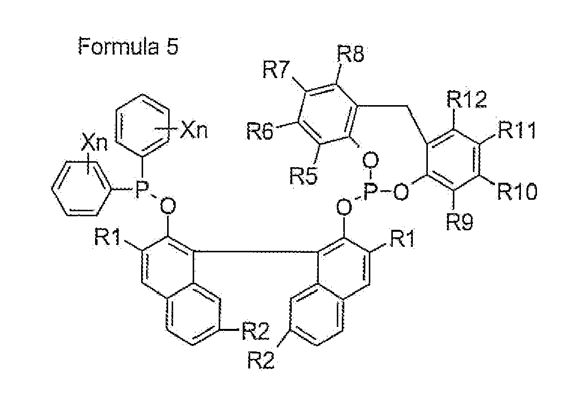 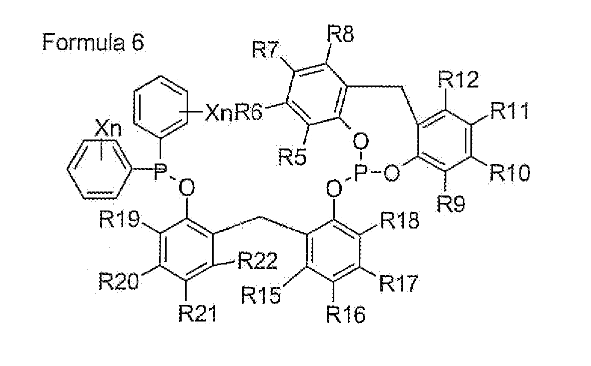 where
R1, R2, R4 are each independently H, an alkyl group having from 1 to 8 carbon atoms, with the proviso that at least one of the R1, R2, R4 groups is not H,
R5 to R22 are each independently H, an alkyl group having from 1 to 8 carbon atoms,
R3 is H, methyl or ethyl,
X is F, Cl or CF₃ when n is 1 or 2,
X is H when n is 0_{;}
and mixtures thereof.

2. A phosphinite phosphite I according to claim 1 where R1, R2, R4, R5, R7, R8, R10, R12, R13 are each independently selected from the group consisting of H, methyl, ethyl, n-propyl, isopropyl and t-butyl.

3. The use of a phosphinite phosphite according to claim 1 or 2 as a ligand in transition metal complexes.

4. A transition metal complex comprising a phosphinite phosphite I according to claim 1 or 2 as a ligand.

5. A transition metal complex according to claim 4, wherein the transition metal used is nickel.

6. A process for preparing transition metal complexes according to claim 4 or 5, wherein an elemental transition metal or a chemical compound comprising a transition metal is reacted with a phosphinite phosphite of the formula I I according to claim 1 or 2.

7. The use of transition metal complexes according to claim 4 or 5 as a catalyst.

8. The use according to claim 7 as a catalyst for the addition of hydrocyanic acid to an olefinic double bond.

9. The use according to claim 7 as a catalyst for the isomerization of organic nitriles.

10. A process for adding hydrocyanic acid to an olefinic double bond in the presence of a catalyst, wherein the catalyst used is a transition metal complex according to claim 4 or 5.

11. The process according to claim 10, wherein hydrocyanic acid is added to butadiene to obtain a compound selected from the group consisting of 2-methyl-3-butenenitrile and 3-pentenenitrile.

12. The process according to claim 10, wherein hydrocyanic acid is added to a 3-pentenenitrile, 4-pentenenitrile or mixtures thereof to obtain adiponitrile.

13. A process for isomerizing organic nitriles in the presence of a catalyst, wherein the catalyst used is a transition metal complex according to claim 4 or 5.

14. The process according to claim 13, wherein 2-methyl-3-butenenitrile is isomerized to 3-pentenenitrile.

## Revendications

1. Phosphinite-phosphites I de formule 1 ou 2 ou 3 ou 4 ou 5 ou 6 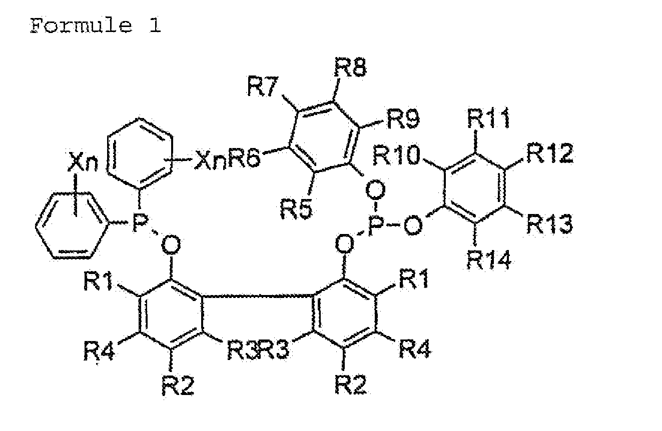 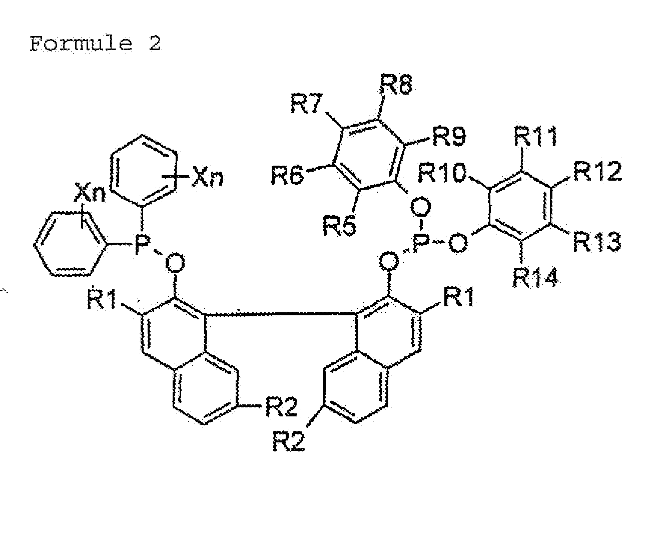 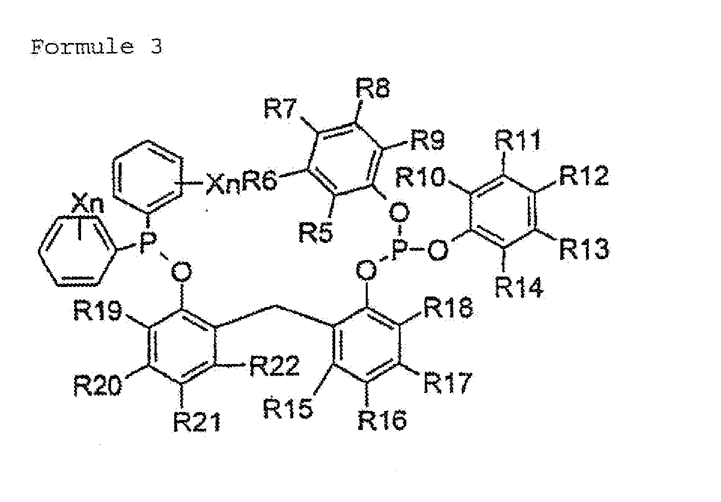 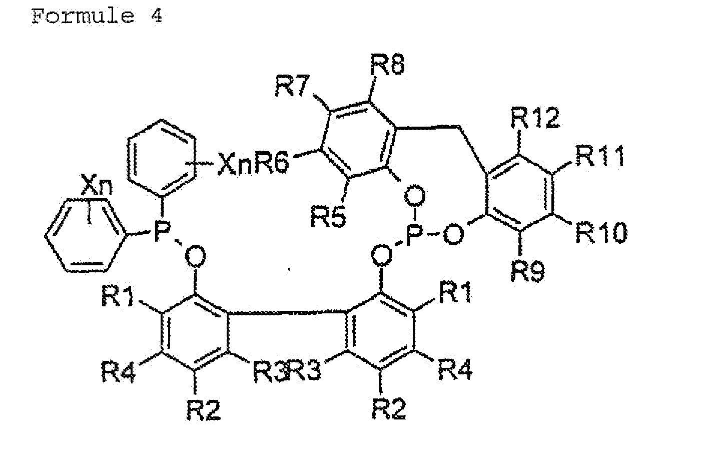 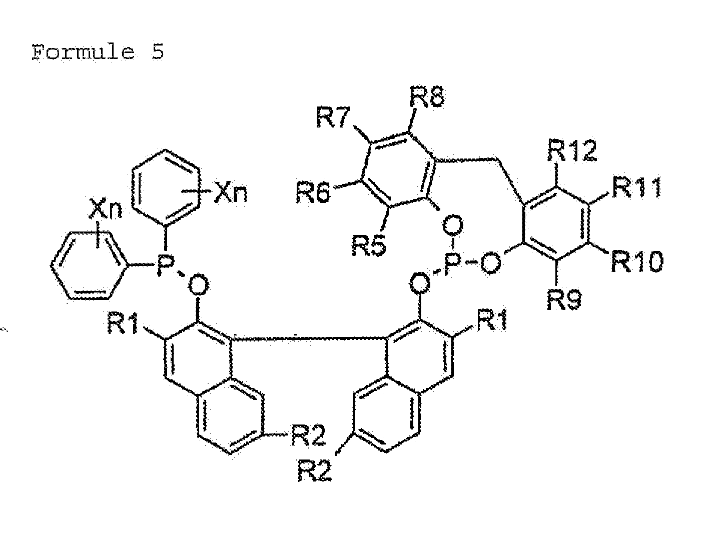 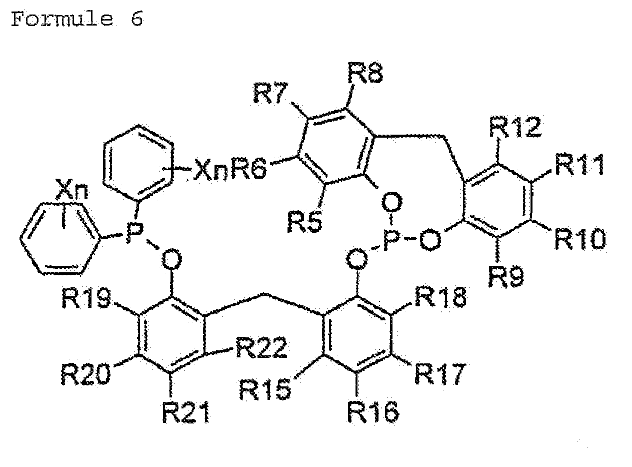 avec
R1, R2, R4 sont indépendamment les uns des autres H, un groupe alkyle contenant 1 à 8 atomes de carbone, à condition qu'au moins un des groupes R1, R2, R4 ne soit pas H,
R5 à R22 sont indépendamment les uns des autres H, un groupe alkyle contenant 1 à 8 atomes de carbone,
R3 est H, méthyle ou éthyle,
X est F, Chou CF₃, lorsque n est 1 ou 2,
X est H, lorsque n est 0
et leurs mélanges.

2. Phosphinite-phosphite I selon la revendication 1, avec R1, R2, R4, R5, R7, R8, R10, R12, R13 choisis indépendamment les uns des autres dans le groupe constitué de H, méthyle, éthyle, n-propyle, i-propyle et t-butyle.

3. Utilisation d'un phosphinite-phosphite I selon la revendication 1 ou 2 en tant que ligand dans des complexes de métal de transition.

4. Complexes de métal de transition contentant en tant que ligand un phosphinite-phosphite I selon la revendication 1 ou 2.

5. Complexes de métal de transition selon la revendication 4, dans desquels le nickel est utilise en tant que métal de transition.

6. Procédé de fabrication de complexes de métal de transition selon la revendication 4 ou 5, **caractérisé en ce qu'**un métal de transition élémentaire ou un composé chimique contenant un métal de transition est mis en rédaction avec un phosphinite-phosphite de formule I selon la revendication 1 ou 2.

7. Utilisation de complexes de métal de transition selon la revendication 4 ou 5 en tant que catalyseur.

8. Utilisation selon la revendication 7 en tant que catalyser pour l'addition d'acide cyanhydrique sur une double liaison oléfinique.

9. Utilisation selon la revendication 7 en tant que catalyseur pour l'isomérisation de nitriles organiques.

10. Procédé d'addition d'acide cyanhydrique sur une double liaison oléfinique en présence d'un catalyseur, dans lequel un complexe de métal de transition salon la revendication 4 ou 5 est utilise en tant que catalyseur.

11. Procédé selon la revendication 10, dans lequel l'acide cyanhydrique est ajouté sur le butadiène, avec obtention d'un composé choisi dans le groupe constitué du 2-méthyl-3-butène-nitrile et du 3-pentène-nitrile.

12. Procédé selon la revendication 10, dans lequel l'acide cyanhydrique est ajouté sur un 3-pentène-nitrile, un 4-pentène-nitrile ou leurs mélanges, avec obtention d'adiponitrile.

13. Procédé d'isomérisation de nitriles organiques en présence d'un catalyseur, dans lequel un complexe de métal de transition selon la revendication 4 ou 5 est utilisé en tant que catalyseur.

14. Procédé selon la revendication 13, dans lequel le 2-méthyl-3-butène-nitrile est isomérisé en 3-pentène-nitrile.
